# EUROPÄISCHE PATENTSCHRIFT

(11) **EP 1 332 219 B1**
(45) Veröffentlichungstag und Bekanntmachung des Hinweises auf die Patenterteilung: **10.05.2006**
(21) Anmeldenummer: 01993480.1
(22) Anmeldetag: 08.11.2001
(51) Int. Cl.: C12N 15/67

(54) **VERWENDUNG DES HUMANEN LRP/MVP PROMOTORS FÜR EINEN THERAPIE-INDUZIERBAREN VEKTOR**
USE OF THE HUMAN LRP/MVP PROMOTOR FOR A VECTOR THAT CAN BE INDUCED BY THERAPY
UTILISATION DES PROMOTEURS LRP/MVP HUMAINS POUR UN VECTEUR INDUCTIBLE PAR THERAPIE

(30) Priorität: 08.11.2000 DE 10055383
(43) Veröffentlichungstag der Anmeldung: 06.08.2003
(73) Patentinhaber: MAX-DELBRÜCK-CENTRUM FÜR MOLEKULARE MEDIZIN, 13125 Berlin (DE)
(72) Erfinder: STEIN, Ulrike, 16341 Schwanebeck (DE); LANGE, Christian, 10439 Berlin (DE); WALTHER, Wolfgang, 16341 Schwanebeck (DE); SCHLAG, Peter, Michael, 13467 Berlin (DE)
(74) Vertreter: Baumbach, Friedrich
(86) Internationale Anmeldenummer: PCT/DE2001/004151
(87) Internationale Veröffentlichungsnummer: WO 2002/038188

(56) Entgegenhaltungen:
- EP-A- 0 657 539
- US-A- 5 968 735
- LANGE CHRISTIAN ET AL: "Cloning and initial analysis of the human multidrug resistance-related MVP/LRP gene promoter." BIOCHEMICAL AND BIOPHYSICAL RESEARCH COMMUNICATIONS, Bd. 278, Nr. 1, 11. November 2000 (2000-11-11), Seiten 125-133, XP002208365 ISSN: 0006-291X
- DATABASE EMBL [Online] ELSEVIER SCIENCE PUBLISHERS, AMSTERDAM, NL; DOE JOINT GENOME INSTITUTE: "Sequencing of Human Chromosome 16" retrieved from EMBL Database accession no. ac009133 XP002208366
- DATABASE EMBL [Online] 6. September 1999 (1999-09-06) STEIN U: "Homo sapiens MVP gene, promoter region" retrieved from EMBL Database accession no. AJ238509 XP002208367
- SCHEFFER G L ET AL: "THE DRUG RESISTANCE-RELATED PROTEIN LRP IS THE HUMAN MAJOR VAULT PROTEIN" NATURE MEDICINE, NATURE PUBLISHING, CO, US, Bd. 1, Nr. 6, 1995, Seiten 578-582, XP002002543 ISSN: 1078-8956

### SEQUENZPROTOKOLL

<110> Max-Delbrück-Centrum für Molekulare Medizin
<120> Verwendung des humanen LRP/MVP-Promoters für einen Therapie-induzierbaren Vektor
<130> PCT/MDC 2000
<140> PCT/DE 01/04151
   <141> 2001-11-08
<150> DE 100 55 383.4
   <151> 2000-11-08
<160> 1
<170> PatentIn Ver. 2.1
<210> 1
   <211> 1903
   <212> DNA
   <213> Homo sapiens
<220>
   <221> promoter
   <222> (1)..(1903)
   <223> humaner LRP/MVP-Promotor
<400> 1
   ggtacctgca gtggcatgat cttggctcac tgcaatctcc acctcctggg ttgaagcgat 60
   tctcctacct cagtctcctg agtagctggg attacaagga cacgtcacca cgcctagcta 120
   atttttgtat tttctagtag agagggggtt tcgccatgtt ggcgaggctg gtcttgaact 180
   cctgacctaa agcttcccaa agtgcctgcc tcagcttccc aaagtgctgg aattacaggc 240
   gtgagctgct gcactcagcc ataaatcgtg tcacttttcc acttaaaatt ttccaaggga 300
   ttccatcttg ccagggaaga gcatgtcaaa ggagaatcca agcgcttttc ccgccacctc 360
   cagttctctg cactcttttt tttgtttttg tttttggcga gtaggggaga cggagtgtag 420
   ctgtgtcacc gaggctggag tgcagtagtg cgatcttggc tcattgcaac ctccacctcc 480
   cgggttcaag cgattctcct gcctcagcct tccaagtagc tgggattaca ggcgcccacc 540
   accacgccca gctaattttt tgtgttggcc aggctggact caaactcctg acctcgagag 600
   ttgcccacct cagcctccca aagtgctggc attacaagcg tgaaccacca tgcccggcct 660
   gcactcttct ttgaacagaa ctctgttctt gtcctggggc ctataccctt gccattcccc 720
   tgcccagtat gttcctccct gttcttcaca ttacctgtgc cttcctgtca atcaagatct 780
   ttgcctctca ccctctctga ggtccaaccc tgaacctcag gtcctccaag acagaggcct 840
   ggggacctgc atgtttaacc agctccccaa gtgatgagtg aggtccaggc aggtatggtg 900
   tacaccacct ccatcccttc catgtatctt accttcctct tctccaggaa gctcagcccg 960
   gagccagaaa cgggaggccc gcctggacaa ggtgctgtcg gacatgaaga gacacaagaa 1020
   gctggaggag cagatccttc gtaccgggag ggacctcttc agcctggact cggaggaccc 1080
   cagccccgcc agccccccac tccgatcctc cgggagtagt ctcttccctc ggcagcggaa 1140
   atactgattc ccactgctcc tgcctctagg gtgcagtgtc cgtacctgct ggagcctggg 1200
   ccctccttcc ccagcccaga cattgagaaa cttgggaaga agagagaaac ctcaagctcc 1260
   caaacagcac gttgcgggaa agaggaagag agagtgtgag tgtgtgtgtg tgttttttct 1320
   attgaacacc tgtagagtgt gtgtgtgtgt tttctattga acacctatag agagagtgtg 1380
   tgtgttttct attgaacatc tatatagaga gagtgtgtga gtgtgtgttt tctattgaac 1440
   acctattcag agacctggac tgaattttct gagtctgaaa taaaagatgc agagctatca 1500
   tctcttaaaa ggaggggctg tagctgtagc tcaacagtta ggccccactt gaagggagag 1560
   gcagaattgt actcacccag attggaaaat gaaagccaga tgggtagagg tgccctcagt 1620
   tagcacctgt cccatctcgg gccctccaac tcctcccagt cccactccag tgcagccagc 1680
   tggctccaag gtagaaaccc atgagcactc agggagcagt gtgccttcag ctgcagcaga 1740
   agcagcccgg aggataaaat gagaaccagc tgcacacggg ccctttaact cccaagcccc 1800
   acccctgggc ttggcctgcc ttgccctgcc gggaagtgat ccccaaggca gggtgagagt 1860
   tccccatctg aggcgtttgt tgcagctacc tgcacttcta gat                   1903

## Beschreibung

### Gegenstand der Erfindung

Gegenstand der Erfindung ist ein Therapie-regulierbares Vektorsystem unter Verwendung des Genpromoters des humanen Lung Resistance Proteins (LRP/MVP). Dieses Vektorsystem zur Therapie-induzierbaren Expression therapeutisch relevanter Gene in Säugerzellen soll in der pharmazeutischen Industrie und der Medizin Anwendung finden.

### Wissenschaftllcher Hintergrund und Beschreibung der Erfindung

Für die Therapie von Tumorerkrankungen werden in wachsendem Maße auch gentherapeutische Strategien verfolgt. Dabei ist es oftmals von Interesse, das therapeutische Gen für einen definierten Zeitraum in ausreichender Menge in den Zielzellen zu exprimieren und für die Tumortherapie wirksam werden zu lassen. Daher sind Promotoren, die durch definierte, Therapie-assoziierte Modalitäten induzierbar sind, von Bedeutung für die Konstruktion konditionell aktiver Vektoren in der Gentherapie. Vektoren, die durch eine bestimmte Therapie regulierbar sind, ermöglichen eine kontrollierte Expression des therapeutischen Gens (Walther 1996, Mol. Biotechnol. 6: 267-86).

Auch aus US-PS 5968735 ist ein Therapieansatz für die Behandlung von Tumoren bekannt, der einen Expressionvektor für Säugerzellen mit der genetischen Information für ein therapeutisch relevantes Protein umfaßt. Die Expression dieses Proteins steht dabei unter Kontrolle eines mdr1 Promotors auf dem gleichen Vektor, der durch Cytostatika induzierbar ist. Durch die Kombination von Cytostatika und der gezielten Expression des therapeutisch relevanten Proteins wird somit eine spezifische Wirkung ermöglicht, die sich direkt gegen Tumorzellen richtet.

Die Erfindung hat das Ziel, die Tumorgentherapie durch therapeutische Modalitäten wie Chemotherapie oder Hyperthermie für den Zeitraum dieser Therapien zu regulieren, so dass die Gentherapie mit der Chemotherapie, Hyperthermie, oder Strahlung zu einer gesteuerten, effizienteren Behandlung der Tumorerkrankung führt.

Das Ziel der Erfindung wird mit Verwendung eines Expreasionsvektors gemäß Anspruch 1 erreicht, durch die Unteransprüche 2-8 wird der erfindungsgemäße Vektor näher gekennzeichnet. Wesentliches Merkmal ist ein Vektor der durch einen Therapie-induzierbaren LRP/MVP Promotor bzw. einer definierten Sequenz davon mit den gleichen Eigenschaften in Bezug auf Induzierbarkeit durch Chemotherapie oder Hyperthermie und einem Gen, welches für ein therapeutisch relevantes Protein kodiert, gekennzeichnet ist. Dieser Promotor ist durch Chemotherapie Hyperthermie oder Strahlung induzierbar und erlaubt so die Therapie-induzierte Expression nachgeschalteter therapeutischer Gene.

Überraschenderweise wurde gefunden, dass der durch die Erfinder isolierte und klonierte humane LRP/MVP-Genpromotor durch Therapie-relevante Faktoren induzierbar ist und die Expression nachgeschalteter Gene somit gesteuert werden kann. Zu diesen Induktoren zählen Chemotherapeutika wie z.B. Cisplatin, 5-Fluoruracil, Adriamycin, Vincristin etc., aber auch weitere Therapie-assoziierte Faktoren, wie z.B. Hyperthermie und Strahlung. Der erfindungsgemäße Vektor gemäß Anspruch 1 ermöglicht damit die Zytostatika- und/oder Hyperthermie-induzierte Expression therapeutischer Gene in den Zielzellen (Tumorzellen) und somit eine Kombination von Gentherapie und Chemotherapie, Hyperthermie und/oder Strahlung.

Als Grundgerüst für die erfindungsgemäßen Vektoren sind alle Konstrukte verwendbar, die für die Expression in Säugerzellen geeignet sind. Diese schließen Vektorgrundgerüste ein, die auf DNA- (z.B. Adenoviren, AAV) oder RNA-Virusvektoren (z.B. MoMuLV, HIV) basieren. Die erfindungsgemäßen Vektoren können demnach mit geeigneten nicht-viralen (Gene-Gun, Liposomen, Elektroporation) oder auch viralen Carriersystemen in die Zielzellen gebracht werden und die therapeutische Wirkung entfalten. Die therapeutische Wirkung des erfindungsgemäßen Vektors entsteht in der Weise, dass nach dem Vektortransfer durch Chemotherapie, Hyperthermie oder Strahlung die Expression des therapeutischen Gens durch den LRP/MVP-Promotor induziert wird und die entstehenden antitumoralen Genprodukte in der Tumorzelle und gegebenenfalls deren Umgebung freigesetzt werden. Mit Hilfe der Erfindung kann somit die Kombination aus Gentherapie (antitumorale Effekte der induzierten therapeutischen Gene) und Chemotherapie, Hyperthermie und/oder Bestrahlung zu einer verbesserten Tumortherapie führen.

Die Erfindung soll durch nachstehende Ausführungsbeispiele näher erläutert werden:
Ausführungsbeispiel 1: Klonierung des humanen LRP/MVP-Genpromoters

### Isolierung des humanen LRP/MVP Promotors

Wir isolierten aus der humanen, multidrug resistenten, aber nicht-P-Glykoproteinexprimierenden Lungenkarzinom-Zelllinie SW1573 eine ca. 1.9 kb große 5'-upstream-Sequenz des LRP-Gens (Anspruch 1). Dazu wurde die genomische DNA aus SW1573-Zellen isoliert und in unterschiedlichen PCR-Varianten einschließlich nested-PCR die Methode des Genome Walking angewendet. Nach Klonierung der ca. 1.9 kb großen 5'-flankierenden Sequenz des LRP-Gens wurden nach der Sequenzanalyse mehrere Konsensus-Elemente für die Bindung von Transkriptionsfatoren identifiziert: so z.B. eine invertierte CCAAT-Box (Y-Box) für die Bindung des im Kontext mit Resistenz beschriebenen Transkriptionsfaktors YB-1, eine E-Box zur Bindung von c-myc, sowie Sequenzen zur Bindung von p53 und SP1.

### Identifizierung des Transkriptionsstarts

Um den Transkriptionsstart zu identifizieren, konstruierten wir mittels der RACE-PCR (rapid amplification of cDNA ends) eine cDNA-Bibliothek der Zelllinie SW1573. Dabei fanden wir einen alternativen 3'-Splice-Ort im Intron 1 im LRP-Gen. Dieses altemative Splicen innerhalb der 5'-untranslatierten Region im Intron 1 führt dann zu zwei unterschiedlichen Varianten LRP-mRNA.

### Nachweis der Promoteraktivität

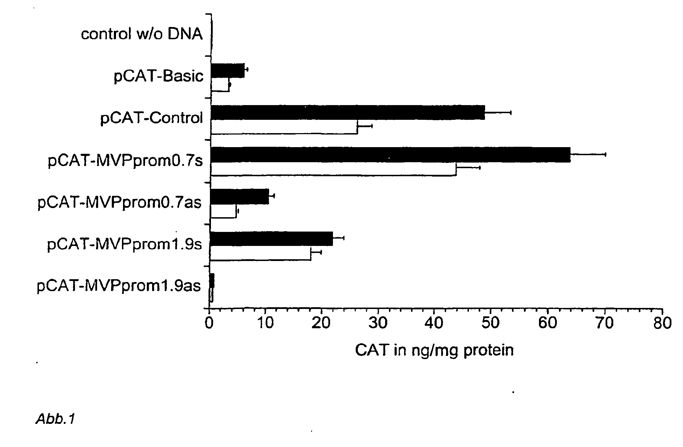

Zur Analyse der Promoteraktivität wurden folgende Konstrukte hergestellt und im Chloramphenikol-Azetyltransferase (CAT)-Assay getestet: pCAT-MVPprom1.9s (beinhaltet die 1.9 kb große Sequenz in sense-Orientierung unter der Kontrolle des CAT-Promoters), pCAT-MVPprom1.9as (in antisense-Orintierung), pCAT-MVPprom0.7s (beinhaltet eine 0.7 kb große Deletionsvariante der 1.9 kb-Sequenz), und pCAT-MVPprom0.7as. Die Konstrukte pCAT-Basic (ohne Promoter) und pCAT-Control (mit SV40-Promoter) sowie die genannten LRP-Promoter-Konstrukte wurden in jeweils in zwei humaneTumorzellinien transfiziert und ein Reportergen-Assay (CAT-ELISA) durchgeführt. Für beide LRP-Promoter-Konstrukte wurden Promoteraktivitäten nachgewiesen, wobei die für pCAT-MVPprom0.7s überrasschenderweise stärker war als für pCAT-MVPprom1.9s und pCAT-Control (Abbildung 1).

### Nachweis der Induzierbarkeit durch Zytostatika

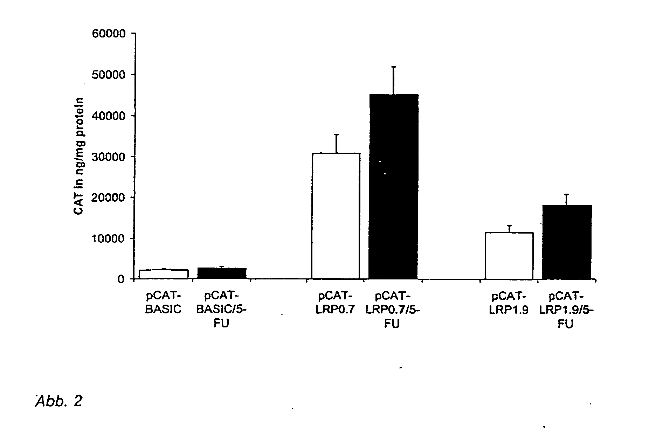

Über die basale Promoteraktivität hinaus kann durch eine Reihe von Zytostatika die LRP/MVP-Promoter-vermittelte Expression nachgeschalteter Gene induziert werden. Nachfolgend ist exemplarisch die 5-Fluoruracil-induzierte CAT-Expression dargestellt: (Abbildung 2)

### Ausführungsbeispiel 2: Konstruktion des Therapie-indizuierbaren Vektorkonstruktes unter Verwendung des LRP/MVP-Genpromoters

### Konstruktionen von Therapie-induzierbaren Vektoren zur Expression von therapeutischen Genen

Der LRP/MVP-Promotor bzw. Teile davon werden in Vektorgrundgerüste in der Weise inseriert, dass der Promotor die Expression eines nachgeschalteten therapeutischen Gens reguliert. Derartige Vektorgrundgerüste können nicht-virale Expressionsvektoren sein (z.B. pcDNA3, pcDNA6 etc.) oder es können virale Vektorgrundgerüste sein (z.B. adenovirale Vektoren, retrovirale Vektoren pLXSN, pLXIN etc.).

### Gentransfer der Vektorkonstrukte

Der Transfer der Vektorkonstrukte kann mit Hilfe nicht-viraler Transfertechnologien erfolgen. Derartige Technologien können die Jet-Injection, die in vivo Elektroporation, das Particle-Bombardment, die Nadelinjektion oder der liposomale Transfer sein (Li, 2000, Gene Ther. 7: 31-4). Alternativ kann bei viralen Vektorkonstrukten über die Herstellung rekombinanter DNA- oder aber RNA-Viruspartikel mit Hilfe geeigneter Helferzellinien der virale Gentransfer für den Transfer in die Zielzellen eingesetzt werden (Walther, 2000, Drugs 60: 249-71). Diese Technologien sind bereits in klinischer Anwendung oder werden perspektivisch für die Behandlung von Krebspatienten eingesetzt.

### Legenden zu den Abbildungen:

### Abb. 1

*Nachweis der LRP*/*MVP-Promoteraktivität im CAT-Assay in den humanen Kolonkarzinom-Zellinien HCT116 (schwarz) und HCT15 (weiß).*

### Abb. 2

*Nachweis der Induzierbarkeit des LRP*/*MVP-Promoters durch 5-Fluoruracil (schwarz) 24 h nach Zytostatika-Applikation*

## Patentansprüche

1. Therapie-induzierbarer Vektor zur Expression von therapeutischen Genen auf der Basis von Vektorgrundgerüsten, die für die Expression in Säugerzellen geeignet sind, **gekennzeichnet durch** einen **durch** Chemotherapie, Hyperthermie oder Strahlung induzierbaren humanen LRP/MVP-Promotor bzw. einer definierten Sequenz davon mit den gleichen Eigenschaften in Bezug auf Induzierbarkeit **durch** Chemotherapie, Strahlung oder Hyperthermie und einem für ein therapeutisches Protein oder nicht-translatierte RNA kodierendes Gen.

2. Therapie-induzierbarer Expressionsvektor nach Anspruch 1, **gekennzeichnet durch** folgende Sequenz des humanen LRP/MVP-Promotors oder Teilen davon mit den gleichen Eigenschaften in Bezug auf Induzierbarkeit **durch** Chemotherapie, Hyperthermie oder Strahlung (Nummerierung ausgehend vom Transkriptionsstart):

3. Therapie-induzierbarer Expressionsvektor nach Anspruch 1 und 2, **gekennzeichnet durch** Mutationsvarianten wie z.B. Insertionen, Deletionen, und/oder Substitutionen mit den gleichen Eigenschaften in Bezug auf Induzierbarkeit **durch** Chemotherapie, Hyperthermie oder Strahlung.

4. Therapie-induzierbarer Expressionsvektor nach Anspruch 1-3, **gekennzeichnet durch** ein Gen eines Zytokins, Enzyms, Antikörpers, Apoptosegens, Resistenzgens, Anti-Onkogens oder einer nicht-translatierten RNA.

5. Therapie-induzierbarer Expressionsvektor nach Anspruch 1-4, **gekennzeichnet durch** das Gen eines Zytokins, wie z.B. Tumornekrosefaktor alpha, Interferon alpha und gamma, Interleukin 2, 6, 7, 12, Colony Stimulating Faktoren GM-CSF oder G-CSF.

6. Therapie-induzierbarer Expressionsvektor nach Anspruch 1-5, **gekennzeichnet durch** das Gen eines Enzyms, wie das der Herpes Simplex Thymidinkinase, Cytosindesaminase, Nitroreductase, Cytochrom P-450.

7. Therapie-induzierbarer Expressionsvektor nach Anspruch 1-6 zur Herstellung eines Therapeutikums gegen Tumorerkrankungen, **gekennzeichnet durch** einen Expressionsvektor für einen nicht-viralen Gentransfer.

8. Therapie-induzierbarer Expressionsvektor nach Anspruch 1-6 zur Herstellung eines Therapeutikums gegen Tumorerkrankungen, **gekennzeichnet durch** einen DNA- oder RNA-Virusvektor-abgeleiteten Expressionsvektor, wobei sie gegebenenfalls in viralen Partikeln für den viralen Gentransfer verpackt sind.

## Claims

1. Therapy-inducible vector for expressing therapeutic genes based on vector base structures that are suitable for the expression in mammalian cells, **characterized by** a human LRP/MVP promoter inducible by chemotherapy or hyperthermia or a defined sequence thereof with the same properties and a gene encoding for a therapeutic protein or non-translated RNA.

2. Therapy-inducible expression vector according to claim 1, **characterized by** the following sequence of the human LRP/MVP promoter or parts thereof (numbering starts at transcription start):

3. Therapy-inducible expression vector according to claim 1 and 2, **characterized by** mutation variants, such as, for example, insertions, deletions, and/or substitutions.

4. Therapy-inducible expression vector according to claim 1-3, **characterized by** a gene of a cytokine, enzyme, antibody, apoptosis gene, resistance gene, anti-oncogene, or non-translated RNA.

5. Therapy-inducible expression vector according to claim 1-4, **characterized by** the gene of a cytokine, such as, for example, tumor necrosis factor alpha, interferon alpha and gamma, interleukin 2, 6, 7, 12, colony stimulating factors GM-CSF or G-CSF.

6. Therapy-inducible expression vector according to claim 1-5, **characterized by** the gene of an enzyme, such as that of herpes simplex thymidine kinase, cytosine deaminase, nitroreductase, cytochrome P-450.

7. Therapy-inducible expression vector according to claim 1-6 for producing a therapeutic agent, **characterized by** an expression vector for non-viral gene transfer.

8. Therapy-inducible expression vector according to claim 1-6 for producing a therapeutic agent **characterized by** an expression vector, derived from a DNA- or an RNA-virus vector, whereby these, as the case may be, may be packaged in viral particles for the viral gene transfer.

## Revendications

1. Vecteur inductible en thérapie pour l'expression de gènes thérapeutiques sur la base de structures fondamentales de vecteur qui sont appropriées à l'expression dans des cellules de mammifères, **se caractérisant par** un promoteur LRP/MVP humain inductible par chimiothérapie, hyperthermie et d'autres moyens ou bien une séquence définie du tel avec les mêmes caractéristiques en rapport avec l'inductibilité par chimiothérapie, hyperthermie et avec un gène codant une protéine thérapeutique ou un ARN non translaté

2. Vecteur inductible en thérapie selon la revendication 1, **se caractérisant par** la séquence suivante du promoteur LRP/MVP humain ou parties du tel (numérotation partant du départ de la transcription):

3. Vecteur d'expression inductible en thérapie selon la revendication 1 et 2, **se caractérisant par** des variantes mutationnelles telles que les insertions, les délétions et/ou les substitutions.

4. Vecteur d'expression inductible en thérapie selon la revendication 1-3, **se caractérisant par** un gène d'une cytokine, d'une enzyme, d'un anticorps, d'un gène de l'apoptose, d'un gène résistant, d'un anti-oncogène ou d'un ARN non translaté.

5. Vecteur d'expression inductible en thérapie selon la revendication 1-4, **se caractérisant par** le gène d'une cytokine, telle que le facteur alpha de la nécrose tumorale, l'interféron alpha et gamma, l'interleukine 2, 6, 7, 12, les facteurs de croissance Colony Stimulating GM-CSF ou G-CSF.

6. Vecteur d'expression inductible en thérapie selon la revendication 1-5, **se caractérisant par** le gène d'une enzyme, tel que celui de la thymidine-kinase Herpes Simplex, la cytosine-désaminase, la nitro-réductase, le cytochrome P-450.

7. Vecteur d'expression inductible en thérapie selon la revendication 1-6 pour la fabrication d'un médicament, **se caractérisant par** un vecteur d'expression pour un transfert génétique non viral.

8. Vecteur d'expression inductible en thérapie selon la revendication 1-6 pour la fabrication d'un médicament, **se caractérisant par** un vecteur d'expression dérivé d'un vecteur viral ADN ou ARN, étant emballés le cas échéant dans des particules virales pour le transfert génétique viral.
